# EUROPEAN PATENT SPECIFICATION

(11) **EP 2 658 460 B1**
(45) Date of publication and mention of the grant of the patent: **22.04.2015**
(21) Application number: 11810804.2
(22) Date of filing: 23.12.2011
(51) Int. Cl.: A61B 17/72

(54) **Intramedullary nail with shape memory elements**
Marknagel mit Formgedächtniselementen
Clou intra-médullaire à éléments de mémoire de forme

(30) Priority: 31.12.2010 EP 10197452
(43) Date of publication of application: 06.11.2013
(73) Proprietor: Orthofix S.r.l., 37012 Bussolengo (VR) (IT)
(72) Inventor: COATI, Michele, 37029 San Pietro in Cariano (Verona) (IT); BAGNASCO, Mara, 20134 Milano (IT); ROSSI, Luigi, 37019 Peschiera del Garda (Verona) (IT); MARINI, Graziano, 37060 Castel d'Azzano (Verona) (IT); ROSSI, Graziano, 37139 (Verona) (IT)
(74) Representative: Botti, Mario
(86) International application number: PCT/EP2011/006552
(87) International publication number: WO 2012/089331

(56) References cited:
- WO-A1-2005/094706
- DE-U1-202005 020 788
- US-A- 6 120 504

## Description

### Field of application

The present invention refers, in its most general aspect, to an intramedullary nail suitable to be inserted into a fractured elongated bone, for example a femur or a tibia, and comprising a cannulated rod extending between a proximal end and a distal end.

More particularly, the invention relates to a nail comprising:
- a cannulated rod extending between a proximal end and a distal end;
- an outside tubular sleeve for receiving said rod, the rod being coaxial with the sleeve and axially guided inside the tubular sleeve;
- shape-memory elements housed in corresponding seats on said rod; each of the elements being able to assume a configuration wherein it is retractably housed in its respective seat, so as to allow the insertion of the nail in the bone, and another configuration wherein said elements project from slots in said sleeve.

### Prior art

Intramedullary nails are known in the field and used in surgical interventions, where they are inserted into a fractured elongated bone to return consistency to the bone and allow the mechanism of bone callus regeneration to take place correctly

These nails comprise a stem or rod having a cylindrical shape and they are either solid or hollow; in the latter case the rod is cannulated.

In order to fix the intramedullary nail to the bone portions that are to be reconstructed, usually two offset holes are provided on the nail, with axes lying on parallel planes and extending diametrically across the rod in correspondence with the distal end of the nail. Generally two further offset holes are provided in correspondence with the proximal end of the nail, with axes lying on parallel planes.

These holes are intended to receive bone screws inserted into the bone after the appropriate holes have been drilled in the bone, in this manner fastening the intramedullary nail inside the fractured bone.

Although still widely used these commonly known nails have known drawbacks as a result of the fact that holes have to be drilled for the insertion of the bone screws. These holes have to be in correspondence with the holes of the inserted intramedullary nail, but since the nail holes are not visible, X-ray techniques are needed, causing cumulative exposure of the operating staff and great inconvenience during the surgical operation.

More recently alternative nail structures have been proposed. European patent No. EP 1 740 113 owned by the same Applicant, for instance, discloses an intramedullary nail that can be inserted into a fractured elongate bone, comprising a straight stem extending between a proximal end and a distal end and further comprising a plurality of elements made of at least a shape-memory material. The free end of these shape-memory elements are positioned on the outside of the stem for fixation of the nail to the bone.

Whilst advantageous from many points of views, such nails with shape-memory elements also have some drawbacks that are still to be overcome.

For instance, due to the absence of bone screws, there is no complete stability of the nail inside the medullary canal and against the high axial stresses caused by a patient's body weight. When this kind of stress is applied, the nail becomes unstable and may even compromise the process of osteosynthesis or cause anomalies in the healing process.

A further prior art solution is disclosed in another European application No. EP 2 133 034, owned by the same applicant. This further prior art discloses an intramedullary nail to be inserted into a fractured long bone comprising a cannulated rod coaxially inserted into a tubular sleeve; a transversal hole is formed at the proximal end of the rod and the sleeve to receive a stop screw holding the nail in a stable position within the medullary canal.

This solution simplifies the structure of nails comprising only shape-memory material, but it remains a compromise that requires surgery in order to inserting the stop screw that stabilizes the nail inside the bone. WO 2005/094 706 A1 discloses an intramedullary nail according to the preamble of claims 1 and 10.

The technical problem of the present invention is that of providing an intramedullary nail with structural and functional features that overcome all the drawbacks of the prior art solutions through a simpler nail construction for housing the shape-memory elements.

Another object of the nail of the present invention is that of providing a nail that does not require bone screws to be stabilized inside the medullary canal.

A further object of the present invention is that of avoiding any danger of bone asymmetry and rotation of the limb during the period in which the nail is inside the bone.

### Summary of the invention

The idea at the basis of the present invention is that of providing an intramedullary nail to be inserted into a fractured elongated bone according to claims 1 and 10.

The dependent claims outline preferred and particularly advantageous embodiments of intramedullary nail according to the invention.

Further features and advantages of the intramedullary nail according to the invention shall become clearer from the following description of an example embodiment thereof, given by way of illustrative and nonlimiting purposes and with reference to the attached drawings.

### Brief description of the drawings

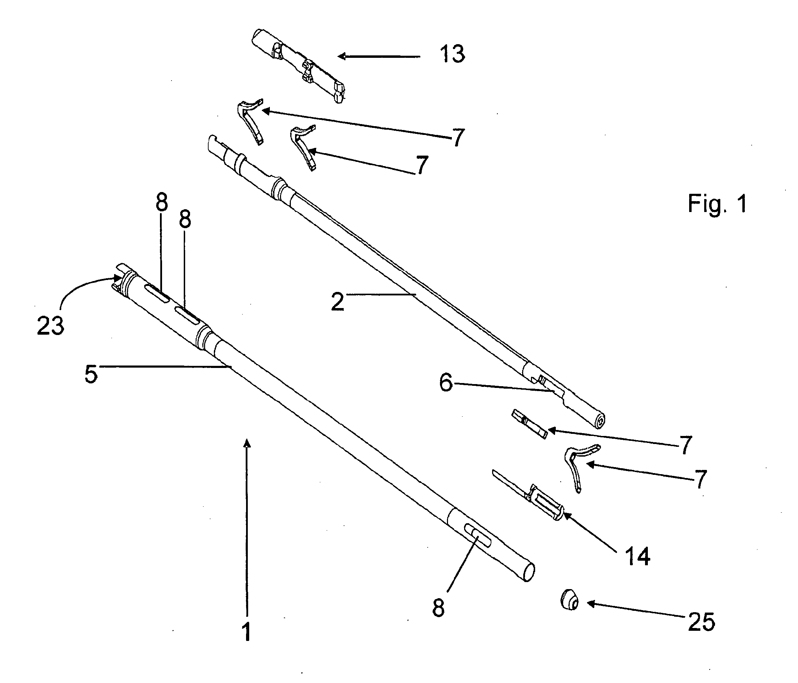

Figure 1 illustrates a perspective view of a nail according to the invention split into its components;

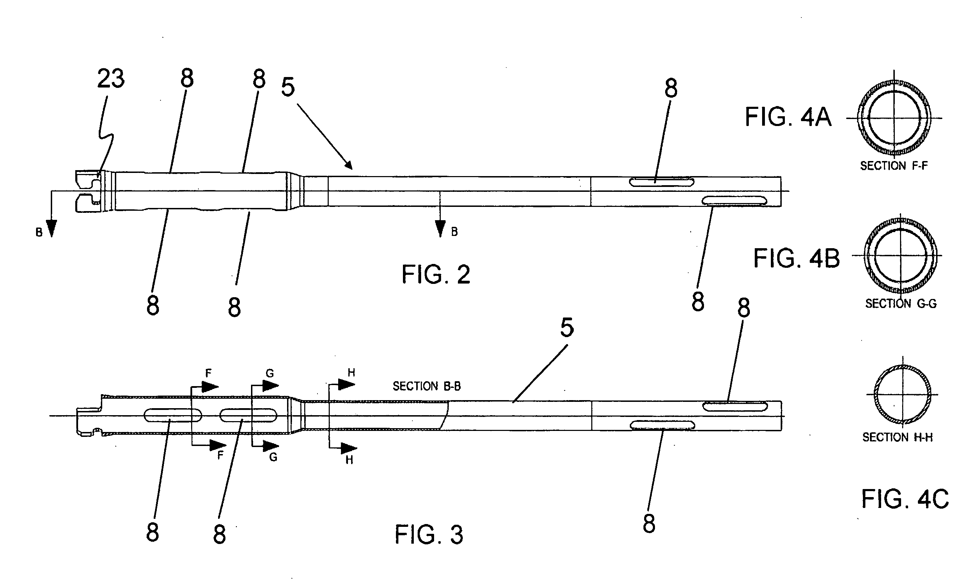

Figure 2 illustrates a side view of a tubular sleeve of the nail of figure 1;
Figure 3 illustrates a partial longitudinal cross section of the tubular sleeve of figure 2 taken along the line B-B;
Figures 4A-4C illustrate views of different transversal cross sections of the tubular sleeve of figure 2 taken along the lines F-F; G-G and H-H respectively;
Figures 5A-5D illustrate front, side, top and cross-section views of a shape-memory element of the nail of the present invention;

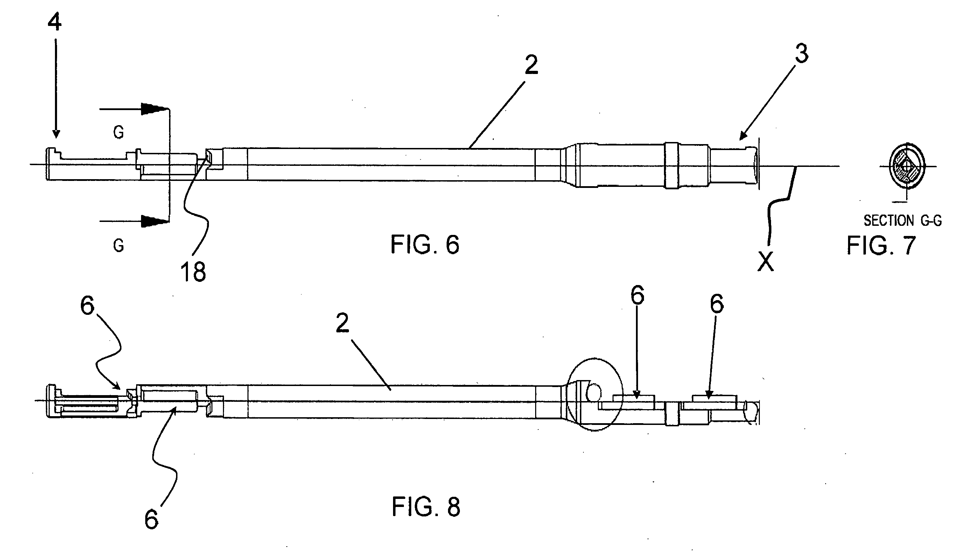

Figure 6 illustrates a side view of a cannulated rod of the nail of figure 1;
Figure 7 illustrates a transversal cross section of the cannulated rod of figure 6 taken along the line G-G;
Figure 8 illustrates a side view of the cannulated rod of figure 6 rotated about the longitudinal axes by 90°;

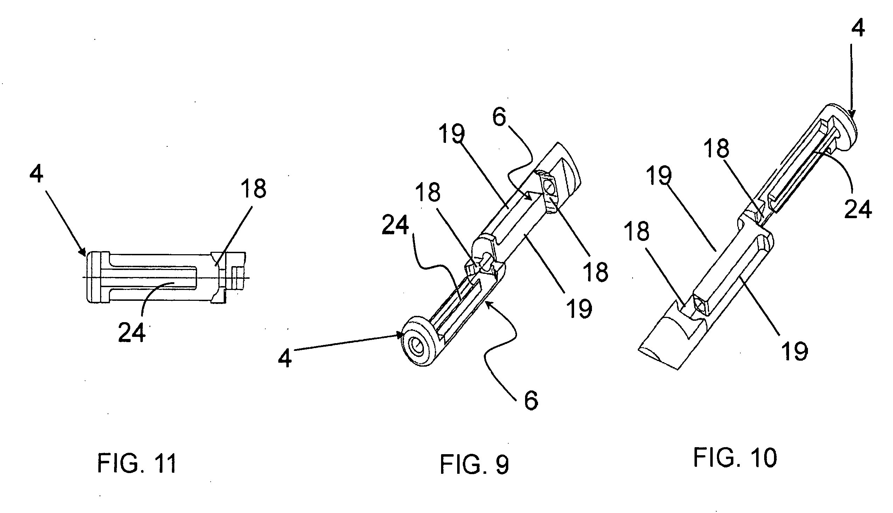

Figure 9 illustrates a perspective view of a detail of a distal end portion of the cannulated rod of figure 6 with seats for the shape-memory elements of figures 5A-5D;
Figure 10 illustrates a perspective view of the distal end of figure 9 in a reversed position;
Figure 11 is a side view of the top portion of the distal end of figures 9 and 10;

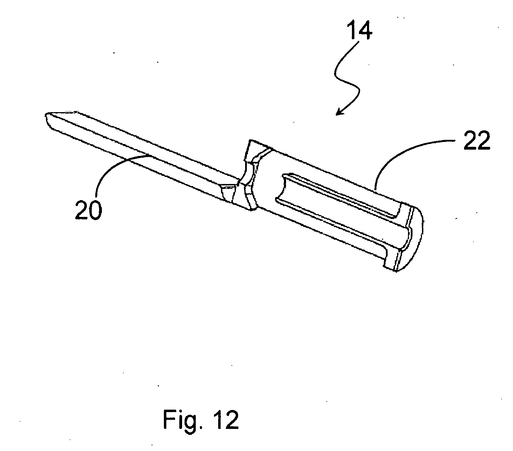

Figure 12 is a perspective view of a distal cover for the cannulated rod;

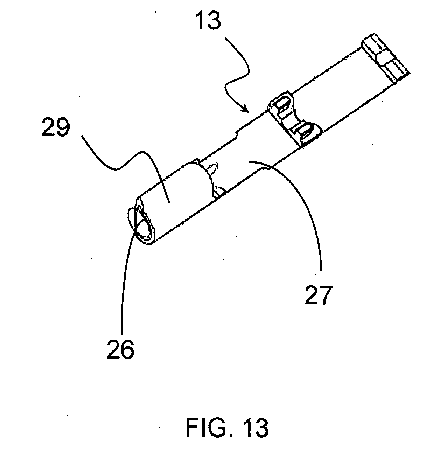

Figure 13 is a perspective view of a proximal cover for the cannulated rod;

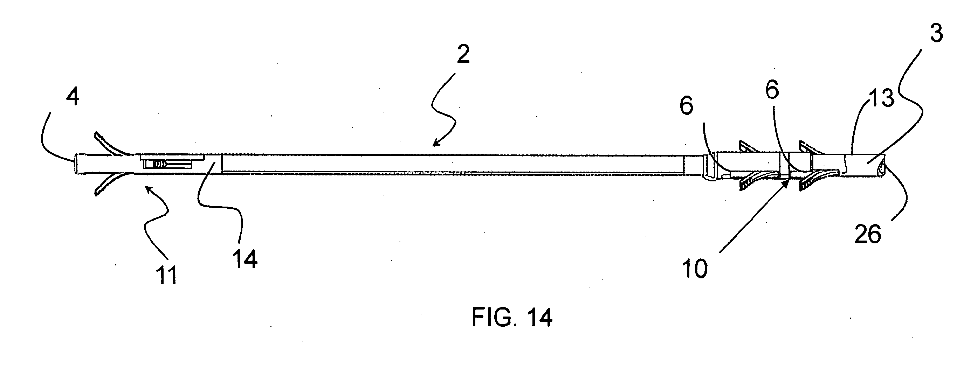

Figure 14 is a side view of the nail of the present invention in its assembled configuration.

### Detailed description

With reference to the aforementioned figures, 1 generally indicates an intramedullary nail in accordance with the present invention, intended to be inserted into a fractured long bone, for instance a femur.

In case of a femoral nail, the nail 1 is provided with an anatomic shape having a proximal 5° curvature to follow the shape of the femoral bone. In other words, the nail 1 and its main components are curved or bent so that the proximal end forms a small angle of 5° with the distal end.

The nail 1 comprises an inner rod 2 extending between a proximal end 3 and a distal end 4.

The nail 1 further comprises an outside tubular sleeve 5 housing the rod 2 that is inserted coaxially into the tubular sleeve 5. Preferably, the nail 1 and its main components, the rod 2 and the sleeve 5 are cylindrical.

The rod 2 is cannulated, that is to say it has an internal cavity that extends along a longitudinal axis, indicated by X, for at least a portion. The rod may be cannulated along its entire length, as a feature that is used to house a guide wire, for example a Kirschner wire, for use during the insertion in the bone medullary canal.

In accordance with the present invention the inner rod 2 is housed slidingly inside the tubular sleeve 5. More specifically, the rod 2 is axially movable inside the sleeve 5 and driven by an external device attached to the proximal end of the nail 1. A relative rotation of the rod inside the sleeve could be realized according to needs. What is important is that the guided movement of the rod 2 inside the sleeve 5 guarantees that active elements 7 housed along the rod are placed in correspondence to slots 8 in the sleeve 5.

In a head portion of the rod 2 a threading 26 is provided for a threaded connection with a suitable driving tool, not shown, for gripping the rod 2 of the nail 1.

In order to allow easy insertion of the nail 1 in the medullary canal an external instrument is foreseen that may be attached to the proximal end of the tubular sleeve 5. To make this connection possible, the proximal end of the tubular sleeve 5 comprises an attachment system 23, for instance of the bayonet type; however, any other fastening system known in the field can be used.

The rod 2 comprises some seats 6 for receiving active elements 7 made of at least a shape-memory material.

The active elements 7 are preferably identical to each other.

Each element 7 has substantially a fork-like shape with two little wings 15 connected by a central portion or core 16.

The seats 6 are in the form of a passing opening 18 for housing the core 16 of the active elements 7 and two lateral recesses 19 for housing the wings 15 of the active elements when in the retracted position.

Because of their shape-memory feature the elements 7 can assume different configurations, from a first configuration in which they are fully hidden inside their seat 6 to an extended configuration in which they protrude from the seats of the rod projecting from the corresponding slots 8 provided in the sleeve 5.

In other words, the active elements 7 are structured and suitable to take a first shape or configuration, in which each elements 7 is retractably housed in its respective seat 6, with the wings 15 housed by the recesses 19, so as to allow the insertion of the nail into the bone. Another shape or configuration is taken when said wings 15 of the active elements 7 project from the respective recesses 19 of the seats 6 to abut against and grip the internal wall of the medullary canal of the fractured bone.

With the term "shape-memory material" we mean a material, known in the art, that has a given starting shape and that takes, under predetermined external conditions or when undergoing a predetermined activation condition, also called "material instruction", a given new shape but that returns to the initial shape when the material instructions are deactivated.

For the meaning of the present invention, the starting shape may correspond to the configuration in which the shape-memory elements 7 are arranged projecting from the seats 6 of the rod 2. However, according to the material used, the starting shape may also correspond to the configuration in which the elements 7 are retracted and housed into the seats.

It must be noted that, while the shape-memory elements 7 may be realized with shape memory alloy, it is preferable to use SIM materials, i.e. materials sensible to Stress-Induced Martensite, for instance Nitinol, an alloy of Nickel and Titanium.

Another characteristic of the shape-memory material lies in the fact that the transition from the first to the second shape, or configuration, is reversible, i.e. the shape-memory elements can change from the second to the first shape, or configuration, allowing the extraction of the nail from the bone.

The nail 1 of the invention is structured with a pair 10 of elements 7 located at the proximal end 3 of the nail and lying on the same plane, therefore having the wings extending on such a plane. We will call this first pair 10 of elements 7 the proximal pair.

Another pair 11 of elements 7 is provided at the distal end 4 of the nail 1. We will call this second pair 11 of elements 7 the distal pair.

Advantageously, the plane on which one shape-memory element 7 of the distal pair 11 lies, is oriented at +45° with respect to the plane of the wings 15 of the shape-memory elements 7 of the proximal pair 10.

The other element of the distal pair 11 is oriented at -45° with respect to the plane of the wings 15 of the shape-memory elements 7 of the proximal pair 10.

Therefore, the first and second element 7 of the distal pair 11 are angularly spaced by 90° from one another.

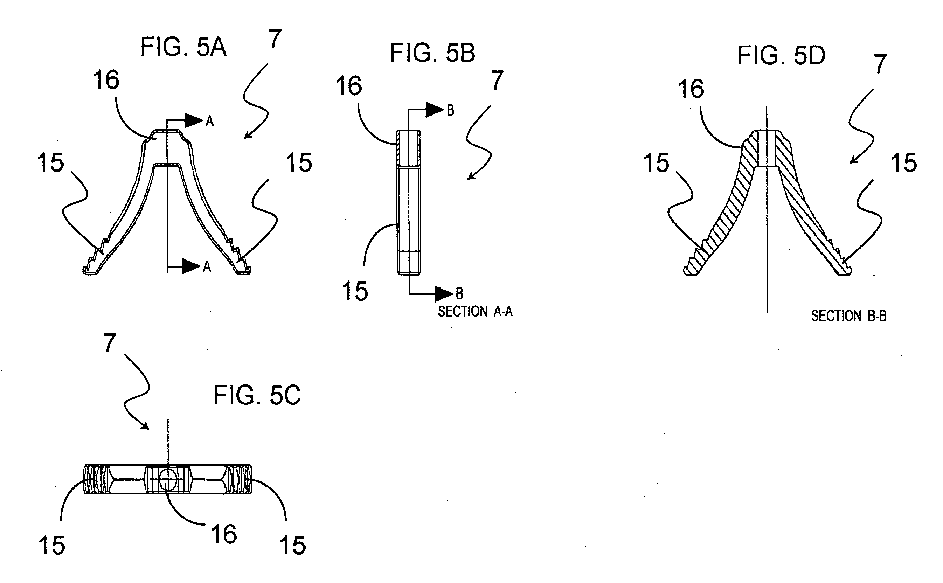

The pane of the wings 15 is the plane on which lie the two wings; for example the section plane of Fig. 5D.

The fact that the distal elements 7 are offset with one another, for example with an offset of 90° sexagesimal, ensures a determined stability on orthogonal planes and it is useful for granting the nail 1 a better grip inside the medullary canal.

Advantageously, the shape-memory elements of the present invention are structurally independent from the rod 2 and are housed in their corresponding seats 6 of the rod but firmly kept in these seats by a cover. The central core 16 of each element 7 is firmly kept in its seat 6 by the cover, while the wings 15 of the elements 7 are freely movable through the openings between the cover 12 and the rod 2 and through the slots 8.

A proximal cover 13 is provided for the proximal pair 10 of shape-memory elements 7 and a distal cover 14 is provided for the distal pair 11 of shape-memory elements 7.

The proximal cover 13 and the distal cover 14 have a particular configuration that cooperate respectively with the proximal end of the rod and the distal end of the rod to be covered.

Each cover 13 or 14 is fixed to the corresponding proximal or distal end portion of the uncovered rod by soldering or other fixing techniques.

The covers 13 and 14, once fixed to the rod, might be considered forming a common piece forming the rod 2 so that each seat 6 is formed by an opening 18 for receiving the core 16 of a shape-memory element 7 and by a couple of opposite recesses 19 for receiving each wing 15 of the same shape-memory element 7 when in the retracted configuration.

In more detail, the distal cover 14 is formed by two portions 20 and 22 that are connected at their respective end forming a single piece and each portion lies on a respective plane which is perpendicular with respect to the other.

Both portions 20, 22 have a concave outside surface that continues the cylindrical surface of the rod 2 when the distal cover is mounted and fixed to the rod 2 to keep the distal pair 11 of shape-memory elements 7 in position.

One of the two portions, numbered 20, of the distal cover 14 is internally flat while the other portion 22 has an elongated internal element having a shape conformed to cooperate with a corresponding internal element 24 provided on the distal end 4 of the rod 2.

The coupling between the distal cover 14 and the distal end of the rod 2 guarantees the continuity of the cylindrical external surface of the cannulated rod 2.

The proximal cover 13 is formed by an elongated portion 27 integrally formed with a short tubular top portion 29 including an internal thread 26 corresponding to the thread formed internally at the proximal end of the rod 2.

The external surface of the elongated portion 27 forms part of the outside concave surface of the proximal portion 3 of the rod 2.

To facilitate the insertion of the nail 1 into the medullary canal of the fractured bone, the tip portion of the sleeve 5 is preferably rounded so as to allow the nail to slide smoothly into said medullary canal.

More specifically, a structurally independent rounded tip 25 is provided to be fixed to the distal end of the sleeve 5.

The tip 25 is soldered to the distal end of the sleeve 5 once the rod 2 has already been inserted into the sleeve.

It must be noted that such a tip 25 maintains the opening of the cannulated rod 2, which has an open end.

As can be appreciated from what has been described, the intramedullary nail according to the present invention meets the requirements and overcomes the drawbacks mentioned in the introductory part of the present description with reference to the prior art.

A clear advantage of the nail according to the present invention is the fact that no screws are needed to stabilize the nail in the medullary canal.

Another advantage of the use of shape-memory elements is that these are structurally independent from the nail rod, which makes it possible to manufacture the rod of a non-shape-memory material, which means a substantial reduction of the production cost.

Of course, a person skilled in the art can apply numerous modifications and variants to the intramedullary nail described above, in order to satisfy contingent and specific requirements.

The scope of protection of the invention is defined by the following claims.

## Claims

1. Intramedullary nail to be inserted into a fractured elongated bone and comprising:
a cannulated rod (2) extending between a proximal end (3) and a distal end (4);
an outside tubular sleeve (5) for receiving said rod (2), the rod (2) being coaxial with the sleeve (5) and axially guided inside the tubular sleeve (5);
shape-memory elements (7) housed in corresponding seats (6) of said rod (2); each of the shape-memory elements (7) being able to assume a configuration of rest in which said shape-memory elements (7) are housed in their respective seats (6) to allow the nail to be inserted into the bone, and a configuration of use in which said shape-memory elements (7) are projecting from slots (8) in said sleeve (5); wherein
a proximal pair (10) of said shape-memory elements (7) is provided at the rod proximal end (3) and a distal pair (11) of said elements (7) is provided at the rod distal end (4);
the shape-memory elements (7) of the proximal pair (10) lie on a common first plane and are retained in their corresponding seats (6) by a proximal cover (13);
the shape-memory elements (7) of the distal pair (11) lie on offset planes with respect to said first plane and are retained in their corresponding seats (6) by a distal cover (14), **characterized in that**
the distal cover (14) is formed by two portions (20, 22) that are connected at their respective ends forming a single piece and each portion lies on a respective plane which is perpendicular with respect to the other.

2. Intramedullary nail according to claim 1, wherein both said portions (20, 22) have an outside concave surface that continues the cylindrical surface of the rod (2) when the distal cover is mounted and fixed to the rod (2) to retain the distal pair (11) of shape-memory elements (7).

3. Intramedullary nail according to claim 1 or 2, wherein the shape-memory elements (7) of the distal pair (11) are angularly spaced at 90° from one another.

4. Intramedullary nail according to any one of the previous claims, wherein one of the shape-memory element (7) of the distal pair (11) lies on a plane at +45° with respect to the first plane, while the other shape-memory element (7) of the distal pair (11) lies on a plane at -45° with respect to the first plane.

5. Intramedullary nail according to any one of the previous claims, wherein said shape-memory elements (7) are made of SIM (Stress Induced Martensite) materials.

6. Intramedullary nail according to claim 5, wherein said SIM material is Nitinol.

7. Intramedullary nail according to any one of the previous claims, wherein each shape-memory element (7) has substantially a fork-like shape with two wings (15) connected by a central portion (16).

8. Intramedullary nail according to any one of the previous claims, wherein said proximal cover (13) is formed by an elongated portion (27) integrally formed with a tubular top portion (29) including an internal thread (26) corresponding to a thread formed internally at the proximal end of the rod (2).

9. Intramedullary nail according to claim 8, wherein said elongated portion 27 forms part of the outside concave surface of the proximal portion of the rod 2.

10. Intramedullary nail to be inserted into a fractured elongated bone and comprising:
a cannulated rod (2) extending between a proximal end (3) and a distal end (4);
an outside tubular sleeve (5) for receiving said rod (2), the rod (2) being coaxial with the sleeve (5) and axially guided inside the tubular sleeve (5);
shape-memory elements (7) housed in corresponding seats (6) of said rod (2); each of the shape-memory elements (7) being able to assume a configuration of rest in which said shape-memory elements (7) are housed in their respective seat (6) to allow the nail to be inserted into the bone, and a configuration of use in which said shape-memory elements (7) are projecting from slots (8) in said sleeve (5); wherein
a proximal pair (10) of said shape-memory elements (7) is provided at the rod proximal end (3) and a distal pair (11) of said elements (7) is provided at the rod distal end (4);
the shape-memory elements (7) of the proximal pair (10) lie on a common first plane and are retained in their corresponding seats (6) by a proximal cover (13);
the shape-memory elements (7) of the distal pair (11) lie on offset planes with respect to said first plane and are retained in their corresponding seats (6) by a distal cover (14), **characterized in that**
said proximal cover (13) is formed by an elongated portion (27) integrally formed with a tubular top portion (29) including an internal thread (26) corresponding to a thread formed internally at the proximal end of the rod (2).

11. Intramedullary nail according to claim 10, wherein the shape-memory elements (7) of the distal pair (11) are angularly spaced at 90° from one another.

12. Intramedullary nail according to claim 10 or 11, wherein one of the shape-memory element (7) of the distal pair (11) lies on a plane at +45° with respect to the first plane, while the other shape-memory element (7) of the distal pair (11) lies on a plane at -45° with respect to the first plane.

13. Intramedullary nail according to one of the previous claims 10-12, wherein said shape-memory elements (7) are made of SIM (Stress Induced Martensite) materials.

14. Intramedullary nail according to one of the previous claims 10-13, wherein each shape-memory element (7) has substantially a fork-like shape.

## Patentansprüche

1. Marknagel, der in einen gebrochenen länglichen Knochen einzusetzen ist und Folgendes umfasst:
einen kanülierten Stab (2), der sich zwischen einem proximalen Ende (3) und einem distalen Ende (4) erstreckt, eine äußere röhrenförmige Hülse (5) zum Aufnehmen des Stabes (2), wobei der Stab (2) koaxial mit der Hülse (5) ist und axial innerhalb der röhrenförmigen Hülse (5) geführt ist, Formgedächtniselemente (7), die in entsprechenden Aufnahmen (6) des Stabes (2) aufgenommen sind, wobei jedes der Formgedächtniselemente (7) dazu in der Lage ist, eine Ruhekonfiguration, in der die Formgedächtniselemente (7) in ihren jeweiligen Aufnahmen (6) aufgenommen sind, um zu ermöglichen, dass der Nagel in den Knochen eingesetzt wird, und eine Anwendungskonfiguration, in der die Formgedächtniselemente (7) aus Schlitzen (8) in der Hülse (5) hervorstehen, anzunehmen, wobei ein proximales Paar (10) der Formgedächtniselemente (7) an dem proximalen Stabende (3) bereitgestellt ist und ein distales Paar (11) der Formgedächtniselemente (7) an dem distalen Stabende (4) bereitgestellt ist, die Formgedächtniselemente (7) des proximalen Paares (10) auf einer gemeinsamen ersten Ebene liegen und durch eine proximale Abdeckung (13) in ihren entsprechenden Aufnahmen (6) zurückgehalten werden, die Formgedächtniselemente (7) des distalen Paares (11) auf in Bezug auf die erste Ebene versetzten Ebenen liegen und durch eine distale Abdeckung (14) in ihren jeweiligen Aufnahmen (6) zurückgehalten werden, **dadurch gekennzeichnet, dass** die distale Abdeckung (14) durch zwei Abschnitte (20, 22) gebildet wird, die an ihren jeweiligen Enden verbunden sind, wobei sie ein einziges Teil bilden und jeder Abschnitt auf einer jeweiligen Ebene liegt, die senkrecht in Bezug auf die andere ist.

2. Marknagel nach Anspruch 1, wobei die beiden Abschnitte (20, 22) eine konkave Außenfläche haben, welche die zylindrische Oberfläche des Stabes (2) fortsetzt, wenn die distale Abdeckung angebracht und an dem Stab befestigt ist, um das distale Paar (11) von Formgedächtniselementen (7) zurückzuhalten.

3. Marknagel nach Anspruch 1 oder 2, wobei die Formgedächtniselemente (7) des distalen Paares (11) um 90° winklig zueinander beabstandet sind.

4. Marknagel nach einem der vorhergehenden Ansprüche, wobei das eine der Formgedächtniselemente (7) des distalen Paares (11) auf einer Ebene bei +45° in Bezug auf die erste Ebene liegt, während das andere Formgedächtniselement (7) des distalen Paares (11) auf einer Ebene bei -45° in Bezug auf die erste Ebene liegt.

5. Marknagel nach einem der vorhergehenden Ansprüche, wobei die Formgedächtniselemente (7) aus SIM (spannungsinduziertes Martensit) Werkstoffen hergestellt sind.

6. Marknagel nach Anspruch 5, wobei der SIM-Werkstoff Nitinol ist.

7. Marknagel nach einem der vorhergehenden Ansprüche, wobei jedes Formgedächtniselement (7) im Wesentlichen eine gabelartige Form mit zwei Flügeln (15), die durch einen Mittelabschnitt (16) verbunden sind, hat.

8. Marknagel nach einem der vorhergehenden Ansprüche, wobei die proximale Abdeckung (13) durch einen länglichen Abschnitt (27) gebildet wird, der integral mit einem röhrenförmigen oberen Abschnitt (29) geformt ist, der ein Innengewinde (26) einschließt, das einem Gewinde entspricht, das innen an dem proximalen Ende des Stabes (2) ausgebildet ist.

9. Marknagel nach Anspruch 8, wobei der längliche Abschnitt 27 einen Teil der konkaven Außenfläche des proximalen Abschnitts des Stabes 2 bildet.

10. Marknagel, der in einen gebrochenen länglichen Knochen einzusetzen ist und Folgendes umfasst:
einen kanülierten Stab (2), der sich zwischen einem proximalen Ende (3) und einem distalen Ende (4) erstreckt, eine äußere röhrenförmige Hülse (5) zum Aufnehmen des Stabes (2), wobei der Stab (2) koaxial mit der Hülse (5) ist und axial innerhalb der röhrenförmigen Hülse (5) geführt ist, Formgedächtniselemente (7), die in entsprechenden Aufnahmen (6) des Stabes (2) aufgenommen sind, wobei jedes der Formgedächtniselemente (7) dazu in der Lage ist, eine Ruhekonfiguration, in der die Formgedächtniselemente (7) in ihrer jeweiligen Aufnahme (6) aufgenommen sind, um zu ermöglichen, dass der Nagel in den Knochen eingesetzt wird, und eine Anwendungskonfiguration, in der die Formgedächtniselemente (7) aus Schlitzen (8) in der Hülse (5) hervorstehen, anzunehmen, wobei ein proximales Paar (10) der Formgedächtniselemente (7) an dem proximalen Stabende (3) bereitgestellt ist und ein distales Paar (11) der Formgedächtniselemente (7) an dem distalen Stabende (4) bereitgestellt ist, die Formgedächtniselemente (7) des proximalen Paares (10) auf einer gemeinsamen ersten Ebene liegen und durch eine proximale Abdeckung (13) in ihren entsprechenden Aufnahmen (6) zurückgehalten werden, die Formgedächtniselemente (7) des distalen Paares (11) auf in Bezug auf die erste Ebene versetzten Ebenen liegen und durch eine distale Abdeckung (14) in ihren jeweiligen Aufnahmen (6) zurückgehalten werden, **dadurch gekennzeichnet, dass** die proximale Abdeckung (13) durch einen länglichen Abschnitt (27) gebildet wird, der integral mit einem röhrenförmigen oberen Abschnitt (29) geformt ist, der ein Innengewinde (26) einschließt, das einem Gewinde entspricht, das innen an dem proximalen Ende des Stabes (2) ausgebildet ist.

11. Marknagel nach Anspruch 10, wobei die Formgedächtniselemente (7) des distalen Paares (11) um 90° winklig zueinander beabstandet sind.

12. Marknagel nach Anspruch 10 oder 11, wobei das eine der Formgedächtniselemente (7) des distalen Paares (11) auf einer Ebene bei +45° in Bezug auf die erste Ebene liegt, während das andere Formgedächtniselement (7) des distalen Paares (11) auf einer Ebene bei -45° in Bezug auf die erste Ebene liegt.

13. Marknagel nach einem der vorhergehenden Ansprüche 10 bis 12, wobei die Formgedächtniselemente (7) aus SIM (spannungsinduziertes Martensit) Werkstoffen hergestellt sind.

14. Marknagel nach einem der vorhergehenden Ansprüche 10 bis 13, wobei jedes Formgedächtniselement (7) im Wesentlichen eine gabelartige Form hat.

## Revendications

1. Clou intra-médullaire à introduire dans un os allongé fracturé, et comprenant:
une tige cannelée (2) qui s'étend entre une extrémité proximale (3) et une extrémité distale (4);
un manchon tubulaire extérieur (5) pour recevoir ladite tige (2), la tige (2) étant coaxiale au manchon (5) et étant guidée axialement à l'intérieur du manchon tubulaire (5);
des éléments à mémoire de forme (7) logés dans des sièges correspondants (6) de ladite tige (2); chacun des éléments à mémoire de forme (7) permettant d'assurer une configuration de repos dans laquelle lesdits éléments à mémoire de forme (7) sont logés dans leurs sièges respectifs (6) afin de permettre l'introduction du clou dans l'os, et une configuration d'utilisation dans laquelle lesdits éléments à mémoire de forme (7) font saillie à partir de fentes (8) dans ledit manchon (5), dans lequel
une paire proximale (10) desdits éléments à mémoire de forme (7) est prévue à l'extrémité proximale (3) de la tige, et une paire distale (11) desdits éléments (7) est prévue à l'extrémité distale (4) de la tige;
les éléments à mémoire de forme (7) de la paire proximale (10) se trouvent sur un premier plan commun et sont retenus dans leurs sièges correspondants (6) par un chapeau proximal (13);
les éléments à mémoire de forme (7) de la paire distale (11) se trouvent sur des plans décalés par rapport audit premier plan et sont retenus dans leurs sièges correspondants (6) par un chapeau distal (14), **caractérisé en ce que**
le chapeau distal (14) est formé par deux parties (20, 22) qui sont connectées à leurs extrémités respectives en formant une seule pièce, et chaque partie se trouve sur un plan respectif qui est perpendiculaire à l'autre.

2. Clou intra-médullaire selon la revendication 1, dans lequel les deux parties (20, 22) présentent une surface extérieure concave qui continue la surface cylindrique de la tige (2) lorsque le chapeau distal est monté et fixé sur la tige (2) pour retenir la paire distale (11) d'éléments à mémoire de forme (7).

3. Clou intra-médullaire selon la revendication 1 ou 2, dans lequel les éléments à mémoire de forme (7) de la paire distale (11) sont espacés de façon angulaire à 90° l'un de l'autre.

4. Clou intra-médullaire selon l'une quelconque des revendications précédentes, dans lequel un des éléments à mémoire de forme (7) de la paire distale (11) se trouve sur un plan à +45° par rapport au premier plan, alors que l'autre élément à mémoire de forme (7) de la paire distale (11) se trouve sur un plan à -45° par rapport au premier plan.

5. Clou intra-médullaire selon l'une quelconque des revendications précédentes, dans lequel lesdits éléments à mémoire de forme (7) sont constitués de matériaux SIM (martensite induite par contrainte).

6. Clou intra-médullaire selon la revendication 5, dans lequel ledit matériau SIM est le nitinol.

7. Clou intra-médullaire selon l'une quelconque des revendications précédentes, dans lequel chaque élément à mémoire de forme (7) présente sensiblement une forme de fourche avec deux ailes (15) qui sont connectées par une partie centrale (16).

8. Clou intra-médullaire selon l'une quelconque des revendications précédentes, dans lequel ledit chapeau proximal (13) est formé par une partie allongée (27) qui est intégralement formée avec une partie supérieure tubulaire (29) comportant un filet interne (26) qui correspond à un filet formé intérieurement à l'extrémité proximale de la tige (2).

9. Clou intra-médullaire selon la revendication 8, dans lequel ladite partie allongée (27) fait partie de la surface extérieure concave de la partie proximale de la tige (2).

10. Clou intra-médullaire à introduire dans un os allongé fracturé, et comprenant:
une tige cannelée (2) qui s'étend entre une extrémité proximale (3) et une extrémité distale (4);
un manchon tubulaire extérieur (5) pour recevoir ladite tige (2), la tige (2) étant coaxiale au manchon (5) et étant guidée axialement à l'intérieur du manchon tubulaire (5);
des éléments à mémoire de forme (7) logés dans des sièges correspondants (6) de ladite tige (2); chacun des éléments à mémoire de forme (7) permettant d'assurer une configuration de repos dans laquelle lesdits éléments à mémoire de forme (7) sont logés dans leurs sièges respectifs (6) afin de permettre l'introduction du clou dans l'os, et une configuration d'utilisation dans laquelle lesdits éléments à mémoire de forme (7) font saillie à partir de fentes (8) dans ledit manchon (5), dans lequel
une paire proximale (10) desdits éléments à mémoire de forme (7) est prévue à l'extrémité proximale (3) de la tige, et une paire distale (11) desdits éléments (7) est prévue à l'extrémité distale (4) de la tige;
les éléments à mémoire de forme (7) de la paire proximale (10) se trouvent sur un premier plan commun et sont retenus dans leurs sièges correspondants (6) par un chapeau proximal (13);
les éléments à mémoire de forme (7) de la paire distale (11) se trouvent sur des plans décalés par rapport audit premier plan et sont retenus dans leurs sièges correspondants (6) par un chapeau distal (14), **caractérisé en ce que**
ledit chapeau proximal (13) est formé par une partie allongée (27) qui est formée intégralement avec une partie supérieure tubulaire (29) comportant un filet interne (26) qui correspond à un filet formé intérieurement à l'extrémité proximale de la tige (2).

11. Clou intra-médullaire selon la revendication 10, dans lequel les éléments à mémoire de forme (7) de la paire distale (11) sont espacés de façon angulaire à 90° l'un de l'autre.

12. Clou intra-médullaire selon la revendication 10 ou 11, dans lequel un des éléments à mémoire de forme (7) de la paire distale (11) se trouve sur un plan à +45° par rapport au premier plan, alors que l'autre élément à mémoire de forme (7) de la paire distale (11) se trouve sur un plan à -45° par rapport au premier plan.

13. Clou intra-médullaire selon l'une quelconque des revendications 10 à 12, dans lequel lesdits éléments à mémoire de forme (7) sont constitués de matériaux SIM (martensite induite par contrainte).

14. Clou intra-médullaire selon l'une quelconque des revendications 10 à 13, dans lequel chaque élément à mémoire de forme (7) présente sensiblement une forme de fourche.
